# EUROPÄISCHE PATENTANMELDUNG

(11) **EP 1 555 015 A1**
(43) Veröffentlichungstag der Anmeldung: **20.07.2005**
(21) Anmeldenummer: 04106113.6
(22) Anmeldetag: 26.11.2004
(51) Int. Cl.: A61K 7/42

(54) **Kosmetische und dermatologische Lichtschutzformulierungen**

(30) Priorität: 19.01.2004 DE 102004002998
(71) Anmelder: Beiersdorf AG, 20245 Hamburg (DE)
(72) Erfinder: Mueller, Anja, 23843 Ruempel (DE); Nissen, Bente, 20144 Hamburg (DE); Lerg, Heike, 22303 Hamburg (DE); Sugar, Martin, 20257 Hamburg (DE); Hoop, Kerstin, 25421 Pinneberg (DE); Steinforth, Melanie, 22303 Hamburg (DE)

(57) **Zusammenfassung**

Lichtschutzwirksame kosmetische oder dermatologische Zubereitung, dadurch gekennzeichnet, daß sie
(a) PEG-30-Dipolyhydroxystearat,
(b) mindestens ein Bis-Resorcinyltriazinderivat,
(c) mindestens ein Dibenzoylmethanderivat und
(d) Titandioxidpartikel
enthält.

## Beschreibung

Die vorliegende Erfindung betrifft kosmetische und dermatologische Lichtschutzzubereitungen, insbesondere betrifft sie kosmetische und dermatologische Formulierungen mit erhöhter UV-A-Schutzleistung.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut:

Die sogenannte UV-C-Strahlung mit einer Wellenlänge zwischen 100 und 280 nm wird von der Ozonschicht der Erdatmosphäre absorbiert und findet sich dementsprechend nicht im Sonnenspektrum. Sie ist daher ohne physiologische Bedeutung beim Sonnenbaden.

Der sogenannte UV-B-Bereich liegt zwischen 290 nm und 320 nm. UV-B-Strahlen sind wesentlich für die lang anhaltende Bräunung der Haut verantwortlich, können aber gleichzeitig ein Erythem, einen einfachen Sonnenbrand oder sogar mehr oder weniger starke Verbrennungen verursachen. Auch chronische Lichtschäden, Photodermatosen und Herpes solaris können durch UV-B-Strahlung hervorgerufen werden.

Man hat lange Zeit fälschlicherweise angenommen, daß die langwellige UV-A-Strahlung mit einer Wellenlänge zwischen 320 nm und 400 nm nur eine vernachlässigbare biologische Wirkung aufweist und daß dementsprechend die UV-B-Strahlen für die meisten Lichtschäden an der menschlichen Haut verantwortlich seien. Inzwischen ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist. Auch kann der schädigende Einfluß der UV-B-Strahlung durch UV-A-Strahlung noch verstärkt werden.

So ist es u. a. erwiesen, daß selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen ― die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Die neueren Erkenntnisse über die Wirkung der UV-A-Strahlen auf die Haut haben dazu geführt, daß man den Schutzmaßnahmen für diesen Strahlenbereich nun erhöhte Aufmerksamkeit widmet. Praktisch kein Sonnenschutzprodukt kommt mehr ohne wirksame UV-A-Filterwirkung aus, reine UV-B-Filterpräparate sind selten.

Beim Auftragen eines Sonnenschutzmittels auf die Haut können die ultravioletten Strahlen durch zwei Effekte abgeschwächt werden: zum einen durch Reflexion und Streuung der Strahlen an der Oberfläche von pulverförmigen Feststoffen (physikalischer Lichtschutz) und zum anderen durch Absorption an chemischen Substanzen (chemischer Lichtschutz). Je nachdem, welcher Wellenlängenbereich absorbiert wird, unterscheidet man zwischen UV-B-Filtern (Absorptionsbereich 280 bis 320 nm), UV-A-Filtern (Absorptionsbereich 320 bis 400 nm) und Breitbandfiltern (Absorptionsbereich 290 bis ca. 380 nm).

Zum Schutz gegen UV-B-Strahlung sind zahlreiche Verbindungen bekannt, wobei deren Absorptionsmaximum möglichst um 308 nm liegen sollte, da hier die höchste Erythemwirksamkeit der Sonnenstrahlung vorliegt. Typische UV-B-Filter sind z. B. Derivate des 3-Benzylidencamphers, der 4-Aminobenzoesäure, der Zimtsäure, der Salicylsäure, des Benzophenons sowie auch des 2-Phenylbenzimidazols.

Auch zum Schutz gegen UV-A-Strahlung sind einige Verbindungen bekannt, wie insbesondere Dibenzoylmethanderivate. Allerdings sind Dibenzoylmethanderivate in der Regel nicht photostabil, weshalb kosmetische oder dermatologische Zubereitungen mit einem Gehalt an dieser Substanz zweckmäßigerweise auch bestimmte UV-Stabilisatoren enthalten sollten.

Neben den reinen UV-A- oder UV-B-Filtern gibt es Substanzen, die beide Bereiche abdecken. Zu dieser Gruppe der Breitbandfilter gehören beispielsweise unsymmetrisch substituierte s-Triazin-Verbindungen, wie z. B. das 2,4-Bis-{[4-(2-ethyl-hexyloxy)-2-hydroxy]-phenyl}-6-(4-methoxyphenyl)-1,3,5-triazin (INCI: Bis-Ethylhexyloxylphenol Methoxyphenyl Triazin), bestimmte Benzophenone, wie z. B. das 2-Hydroxy-4-methoxybenzophenon (INCI: Benzophenone 3) oder das 2,2'-Methylen-bis-(6-(2H-benzotriazol-2-yl)-4-(1,1,3,3-tetramethylbutyl)-phenol) (INCI: Methylene Bis-Benzotriazolyl Tetramethylenebutylphenol).

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen. Da zur Charakterisierung einer Filtersubstanz nicht nur die Lage des Absorptionsmaximums, sondern vor allem der Absorptionsbereich wichtig ist, werden von jeder Substanz Absorptionsspektren aufgenommen. Für die Dosierung der Substanzen in den fertigen Formulierungen können die Extinktionswerte aber allenfalls eine Orientierungshilfe bieten, denn durch Wechselwirkungen mit Inhaltsstoffen der Haut oder der Hautoberfläche selbst können Unwägbarkeiten auftreten. Ferner ist in der Regel schwierig vorab abzuschätzen, wie gleichmäßig und in welcher Schichtdicke die Filtersubstanz in und auf der Hornschicht der Haut verteilt ist.

Zur Prüfung der UV-A-Schutzleistung wird üblicherweise die IPD-Methode verwendet (IPD **≡** immediate pigment darkening). Hierbei wird ― ähnlich der Bestimmung des Lichtschutzfaktors ― ein Wert ermittelt, der angibt, um wieviel länger die mit dem Lichtschutzmittel geschützte Haut mit UV-A-Strahlung bestrahlt werden kann, bis die gleiche Pigmentierung auftritt wie bei der ungeschützten Haut.

Die Einsatzkonzentration bekannter Lichtschutzfiltersubstanzen, die insbesondere auch im UV-A-Bereich eine hohe Filterwirkung zeigen, ist allerdings häufig ― gerade in Kombination mit anderen zu lösenden Substanzen ― be grenzt. Um z. B. hohe Mengen an öllöslichen UV-Filtersubstanzen einsetzen zu können, bräuchte man eine sehr große Ölphase (> ca. 35 Gew.-%). Allerdings kann die hydrophobe Phase einer Emulsion ― beispielsweise einer W/O-Emulsion ― selbstverständlich nicht beliebig groß gewählt werden, da auch die Größe der Phasen die Stabilität einer Emulsion entscheidend mitbeeinflußt. Wenn eine große Ölphase (von mehr als etwa 35 Gew.-%) gewünscht ist, müssen daher gemäß dem Stand der Technik Stabilisatoren wie Wachse oder weitere Emulgatoren eingesetzt werden, um eine langzeitstabile Emulsion mit einer Stabilität von mehreren Jahren zu erhalten. Nachteil dieser Vorgehensweise ist allerdings, daß die Emulsionen dadurch relativ fest werden und ― ins besondere auf behaarter Haut - nicht mehr so gut zu verteilen sind.

Eine weitere, nach dem Stand der Technik bekannte Methode, Lichtschutzzubereitungen mit sehr hohen Lichtschutzfaktoren (LSF größer 25) herzustellen, besteht darin, UV-Filtersubstanzen so zu kombinieren, daß sich nicht die gesamte UV-Filtermenge in der Ölphase der Emulsion befindet, was selbstverständlich nur dann möglich ist, wenn auch wasserlösliche UV-Filtersubstanzen eingesetzt werden. Nachteil derartiger Emulsionen, welche wasserlösliche UV-Filtersubstanzen enthalten, ist, daß diese gewöhnlich nur bedingt wasserfest sind.

Der Wasserfestigkeit von Lichtschutzformulierungen ist aber besondere Bedeutung beizumessen, da die meisten Sonnenschutzmittel in Wassernähe oder bei sportlicher Betätigung (Schwitzen) angewendet werden. Ein wasserfestes Sonnenschutzmittel schützt den Anwender nicht nur nach dem Baden, sondern bewahrt ihn auch während des Badens vor einem Sonnenbrand. Es ist ein weitverbreiteter Irrtum, daß Wasser einen guten oder gar ausreichenden Schutz vor ultravioletter Strahlung bietet. Vielmehr haben Untersuchungen gezeigt, daß noch 1 m unter der Wasseroberfläche die Durchlässigkeit für UV-B-Strahlen bei ca. 50 % liegt. Es ist daher ratsam, daß auch Wassersportler, welche z. B. schwimmern, surfern oder schnorcheln, sowie insbesondere Kinder, die oft stundenlang am bzw. im Wasser spielen, die Haut mit einem gut haftenden, durch (Salz-) Wasser und Schweiß nur schwer abspülbaren Sonnenprodukt vor einer zu intensiven und übermäßigen Sonneneinstrahlung schützen.

Im Sinne einer optimalen Wasserfestigkeit wäre daher der Verzicht auf wasserlösliche UV-Filter wünschenswert.

Es war daher Aufgabe der vorliegenden Erfindung, auf einfache und preiswerte Weise zu Zubereitungen zu gelangen, welche eine hohe UV-A-Schutzleistung erreichen und sich gleichzeitig durch gute Wasserfestigkeit und gute Verteilbarkeit auszeichnen.

Es war überraschend und für den Fachmann nicht vorauszusehen, daß eine lichtschutzwirksame kosmetische oder dermatologische Zubereitung, dadurch gekennzeichnet, daß sie
(a) PEG-30-Dipolyhydroxystearat,
(b) mindestens ein Bis-Resorcinyltriazinderivat,
(c) mindestens ein Dibenzoylmethanderivat und
(d) Titandioxidpartikel
enthält,
den Nachteilen des Standes der Technik abhelfen würden.

Die erfindungsgemäßen Zubereitungen stellen in jeglicher Hinsicht überaus befriedigende Präparate dar, welche sehr gute sensorische und kosmetische Eigenschaften zeigen, wie beispielsweise die Verteilbarkeit auf der Haut oder das Einzugsvermögen in die Haut. Sie zeichen sich ferner durch eine sehr gute Lichtschutzeffektivität, eine überaus hohe UV-A-Schutzleistung sowie durch eine ausgezeichnete Hautverträglichkeit bei gleichzeitig hervorragenden Hautpflegedaten aus.

Es ist insbesondere erstaunlich und einer der besonderen Vorteile der vorliegenden Erfindung, daß auf den Einsatz von wasserlöslichen UV-A-Filtern ganz verzichtet werden kann. Weitere Vorteile der vorliegenden Erfindung sind, daß trotz eines hohen Ölphasenanteiles von mehr als 35 Gew.-% (bezogen auf das Gesamtgewicht der Zubereitung) kein weiterer Emulgator bzw. Coemulgator notwendig ist und daß ferner der Anteil an weiteren Stabilisatoren wie Wachsen sehr niedrig gewählt werden kann (weniger als 1,5 Gew.-%, idealerweise weniger als 1 Gew.-%). Auf diese Weise wird trotz einer hohen Ölphase eine fließfähige Formulierung (mit einer Viskosität von <10.000 mPa·s ― bestimm bar mit einem Haake Viskotester VT-02 bei 25 °C) erhalten und somit eine gute Verteilbarkeit und ein optimaler Lichtschutz gewährleistet.

Der besonders hohe UV-A-Schutz gelingt durch die spezielle Kombination der UV-A-Filter aus zwei bei Raumtemperatur (25 °C) festen lipophilen Filtern (Bis-Resorcinyltriazinderivat und Dibenzoylmethanderivat) und einem pigmentären Filter (TiO₂).

Besonders vorteilhafte Zubereitungen im Sinne der vorliegenden Erfindung enthalten mehr als 3 Gew.-%, insbesondere mehr als 10 Gew.-% ― jeweils bezogen auf das Gesamtgewicht der Zubereitung ― an erfindungsgemäßen UV-A-Filtersubstanzen. Es ist ferner erfindungsgemäß vorteilhaft, wenn Titandioxid den höchsten (Gewichts-) Anteil in der erfindungsgemäßen UV-A-Filterkombination hat.

Die erfindungsgemäßen Stoffkombinationen wirken erstaunlicherweise synergistisch, also überadditiv in bezug auf die Einzelkomponenten. Sie sind ohne weitere Zusätze photostabil und zeigen eine überraschend hohe Schutzleistung im UV-A-Bereich.

PEG-30-Dipolyhydroxystearat wird von der Gesellschaft Uniqema unter der Warenbezeichnung ARLACEL® P135 verkauft.

Erfindungsgemäße Bis-Resorcinyltriazinderivate absorbieren im UV-A-Bereich und zeichnen sich durch die folgende Struktur aus: 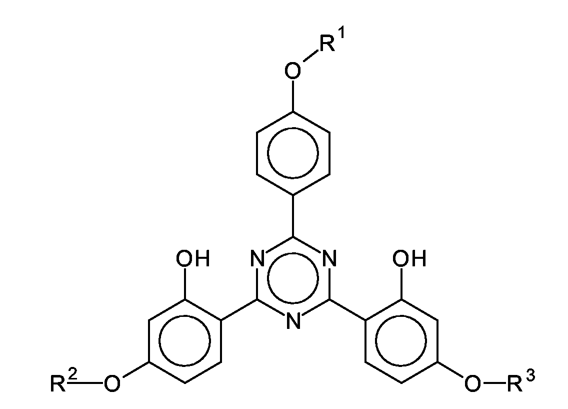 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt ist das 2,4-Bis-{[4-(2-Ethylhexyloxy)-2-hydroxy]-phenyl}-6-(4-methoxyphenyl)-1,3,5-triazin (INCI: Bis-Ethylhexyloxyphenol Methoxyphenyl Triazin), welches unter der Handelsbezeichnung Tinosorb® S bei der CIBA-Chemikalien GmbH erhältlich ist.

Vorteilhaftes Dibenzoylmethanderivat im Sinne der vorliegenden Erfindung ist insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird. Ferner vorteilhaft ist das 2,2'-Methylen-bis-(6-(2H-benzotriazol-2-yl)-4-(1,1,3,3-tetramethylbutyl)-phenol). Das oder die Dibenzoylmethanderivate können vorteillhaft jeweils einzeln oder in beliebigen Kombinationen miteinander eingesetzt werden.

Die Titandioxidpartikel können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Die Titandioxidpartikel können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Erfindungsgemäß vorteilhafte Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |

Die Zubereitungen im Sinne der vorliegenden Erfindung liegen bevorzugt in Form von W/O-Emulsionen vor.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant™ von der Fa. Lonza erhältlich ist), lodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber den noch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Goodrich], beispielsweise Carbopole der Typen 980, 981, 1382, 2984, 5984, ETD 2020, ETD 2050, Ultrez 10, jeweils einzeln oder in Kombination.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.-% bis zu 8 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.
Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs). Es ist ferner vorteilhaft, Wachskomponenten aus der Gruppe der Glyceride, insbesondere aus der Gruppe der Triglyceride zu wählen. Besonders vorteilhaft im Sinne der vorliegenden Erfindung ist C₁₈₋₃₆ Triglycerid, welches unter der Handelsbezeichnung Syncrowax HGLC bei der Croda GmbH erhältlich ist.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Ole yloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 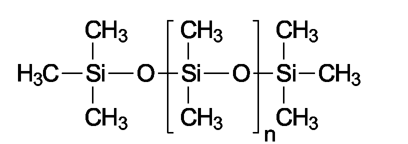 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
wobei die verwendeten Mengenateile so gewählt werden, daß die Menge des Wasserstoffes des Organopolysiloxans (1) oder der ungesättigten aliphatischen Gruppen des Organopolysiloxans (2)
- im Bereich von 1 bis 20 mol-% liegt, wenn das Organopolysiloxan nicht cyclisch ist und
- im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Silikonölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem Rowe *Colour Index,* 3. *Auflage, Society of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calciumund Bariumsalze der 1-(2-Sulfo-4-methyl-1-phenylazo)-2-naphthylcarbonsäure, Calciumsalz der 1 -(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.

Ferner vorteilhaft sind öllösliche Naturfarbstoffe, wie z. B. Paprikaextrakte, β-Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCl)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend können die Zubereitungen im Sinne der vorliegenden Erfindung vorzugsweise weitere UV-A-, UV-B- und/oder Breitbandfiltersubstanz enthalten. Die Formulierungen können, obgleich nicht notwendig, gegebenenfalls auch ein oder mehrere organische und/oder anorganische Pigmente als UV-Filtersubstanzen enthalten, welche in der Wasser- und/oder der Ölphase vorliegen können.

Die erfindungsgemäßen Zubereitungen können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Octyl Salicylate) und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan ― Copolymer welches beispielsweise unter der Handelsbezeichnung Parsol® SLX bei Hoffmann La Roche erhältlich ist.

Bevorzugte weitere anorganische Pigmente sind Metalloxide und/oder andere in Wasser schwerlösliche oder unlösliche Metallverbindungen, insbesondere Oxide Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium- oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicyclo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 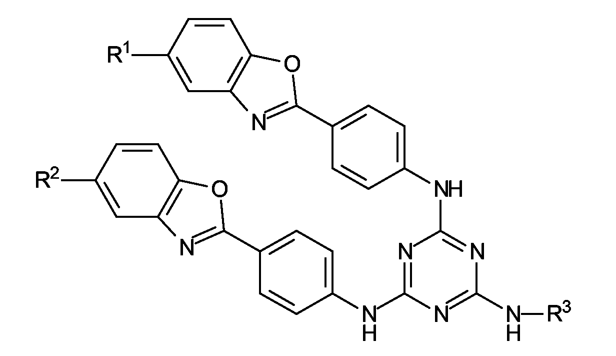 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 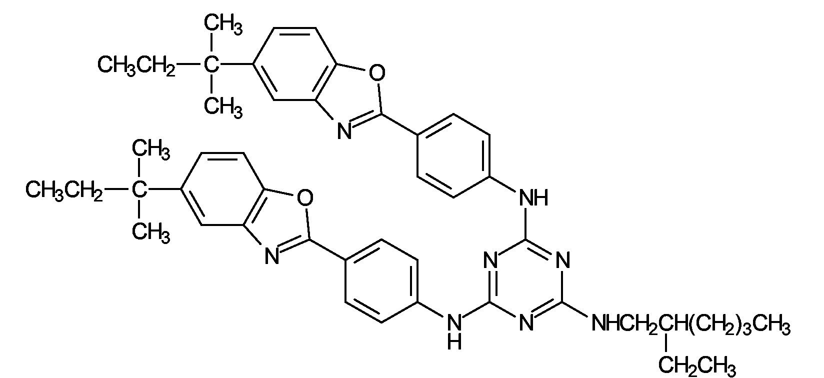 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - bei - spielsweise in Partikelgrößen von 10 nm bis zu 300 nm ― vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 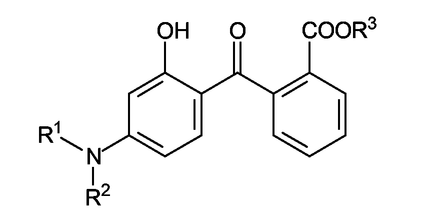 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 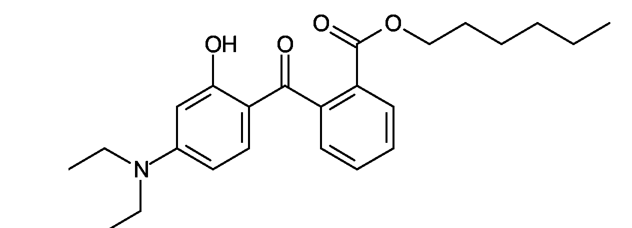 und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoësäure-tris(2-ethylhexylester), auch: 2,4,6-Tris-[anilino-(p-carbo-2'-ethyl-1'-heoyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z.B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2Hbenzotriazol-2-yl)-4-methyl-6-[2-methyl-3-[1,3,3,3-tetramethyl-1 -[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 2,0 Gew.-% bis 40 Gew.-%, vorzugsweise 3,0 bis 35 Gew.-%, insbesondere 5,0 bis 30 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die nachfolgenden Beispiele sollen die vorliegende Erfin dung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### W/O-Emulsionen

| | **1** | **2** | **3** | **4** | **5** |
|---|---|---|---|---|---|
| PEG-30 Dipolyhydroxystearat | 2,00 | 4,00 | 1,00 | 2,00 | 1,00 |
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | 5,00 | | 2,00 |
| Cyclomethicon | 10,00 | 5,00 | | 5,00 | 3,00 |
| UVASorb® K2A | | | | 0,50 | |
| Uvinul® A Plus | | 2,00 | | 4,00 | |
| Bis-Ethylhexyloxyphenol Methoxyphenyl Triazin | 1,00 | 2,50 | 2,00 | 0,50 | 2,50 |
| Drometrizol Trisiloxan | | | | 1,00 | |
| Phenylbenzimidazolsulfonsäure | | | 0,50 | | |
| 4-Methylbenzylidencampher | 4,00 | | | | |
| Methylen Bis-Benztriazolyl Tetramethylbutylphenol | | | | | 3,00 |
| Ethylhexyl Methoxycinnamat | 3,00 | 5,00 | | | 10,00 |
| Diethylhexyl Butamido Triazon | | 1,00 | | | 6,50 |
| Ethylhexyl Triazon | 3,00 | | | | 4,00 |
| Ethylheyl Salicylat | | | 5,00 | | 3,50 |
| Octocrylen | | 5,00 | 1,00 | | 2,00 |
| Butyl Methoxydibenzoylmethan | 3,00 | 4,00 | 1,50 | 3,00 | 4,00 |
| Titandioxid T805 | 5,00 | 4,00 | 2,00 | 6,00 | 6,00 |
| Zinkoxid Z-Cote | 2,00 | | | | 1,00 |
| Dicaprylyl Carbonat | 5,00 | | 15,00 | | 4,00 |
| Isopropyl Stearat | | | 5,00 | 5,00 | |
| Butylenglykol Dicaprylat/Dicaprat | 3,00 | 5,00 | | | 3,00 |
| Dihexyl Carbonat | | 5,00 | | | |
| C12-15 Alkyl Benzoat | 7,00 | | 10,00 | | |
| Mineral Öl | 5,00 | | | | 6,00 |
| Kokosfettsäureglycerid | | 2,00 | | 5,00 | |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Alkohol | 2,00 | | | 5,00 | 1,00 |
| NaOH 45% | | | 0,40 | | |
| Farbstoff, öllöslich | 0,02 | | | | |
| Parfüm | 0,30 | 0,45 | 0,35 | | 0,15 |
| Wassser | ad 100 | ad 100 | ad 100 | ad 100 | ad 100 |

### W/O Emulsionen

| | **6** | **7** | **8** | **9** | **10** | **11** |
|---|---|---|---|---|---|---|
| Cetyldimethicon Copolyol | | | | | 1,00 | |
| PEG-30-dipolyhydroxystearat | 4,00 | 5,00 | 3,00 | 5,00 | 3,00 | 3,50 |
| Bis-Ethylhexyloxyphenol Methoxyphenyl Triazin | 0,5 | 2,00 | 3,00 | 1,50 | 2,50 | 2,00 |
| Butyl Methoxydibenzoylmethan | 2,00 | 3,00 | 4,00 | 1,50 | 1,50 | 4,00 |
| UVASorb® K2A | | | | 2,00 | | |
| Uvinul ® A Plus | | 1,00 | | 3,00 | | |
| Phenylbenzmidazol Sulfonsäure | | | | | 2,00 | |
| Dinatrium Phenyl Dibenzimidazol Tetrasulfonat | | | 1,00 | | | |
| Ethylhexyl Methoxycinnamat | | 8,00 | | | 5,00 | 4,00 |
| Diethylhexyl Butamido Triazon | 3,00 | 1,00 | 2,00 | 1,00 | | 3,00 |
| Ethylhexyl Triazon | | | | 2,00 | 4,00 | |
| Octocrylen | | 2,00 | 3,00 | | 2,00 | 2,50 |
| Drometrizol Trisiloxan | 1,00 | | | | | |
| Titandioxid Uvinul® TiO₂ | 2,00 | 6,50 | 6,00 | 3,50 | 4,00 | |
| Titandioxid MT-100 TV | | | | | | 2,00 |
| Zinkoxid Z-Cote® HP1 | | | | 3,00 | | |
| Mineralöl | 5,00 | | | 5,00 | | 8,00 |
| Kokosfettsäureglycerid | 4,00 | 6,50 | | 5,00 | | |
| C12-15 Alkyl Benzoat | | | 5,00 | 8,00 | 9,00 | |
| Dicaprylylether | 10,00 | 5,00 | 2,00 | | | 7,00 |
| Butylenglycol Dicaprylat/Dicaprat | 6,00 | | 9,00 | 7,00 | 8,00 | 4,00 |
| Cetyl Dimethicon | 0,50 | 1,00 | 0,50 | | | |
| C18-36 Fettsäure Triglycerid | | 1,00 | 0,50 | 1,0 | | |
| Cyclomethicon | 2,00 | 3,00 | 3,00 | | | 2,00 |
| Na Starch Octenylsuccinat | | 0,50 | 0,50 | 1,00 | | |
| PVP Eicosene Copolymer | 0,50 | | | | 1,50 | 1,00 |
| Trinatrium EDTA | 1,00 | | 1,00 | 0,50 | 0,35 | |
| Ethylhexyloxyglycerin | | 0,30 | | | | 0,50 |
| Methylpropandiol | | | | | | 7,50 |
| Glycerin | 5,00 | 7,50 | 6,00 | 8,00 | 7,50 | 2,50 |
| Butylenglykol | | 2,50 | | | | |
| Glycin Soja | | 1,00 | | | | |
| MgSO₄ | 1,00 | 0,50 | 0,30 | 0,30 | 0,50 | |
| Milchsäure & Natriumsalz der Milchsäure | 1,00 | 0,50 | | | | 0,85 |
| Vitamin E | 0,50 | | 0,50 | 1,00 | | 1,00 |
| DMDM Hydantoin | | 0,60 | | | 0,20 | |
| Methylparaben | 0,50 | | | | 0,15 | |
| Phenoxyethanol | 0,50 | 0,40 | 0,50 | 0,60 | 1,00 | 0,60 |
| Dihydroxyaceton | | | | | 5,50 | |
| Alkohol | 3,00 | | 2,00 | 3,00 | | 1,00 |
| Parfüm | 0,20 | | 0,20 | 0,20 | | 0,20 |
| Wasser | ad 100 | ad 100 | ad 100 | ad 100 | ad 100 | ad 100 |

## Patentansprüche

1. Lichtschutzwirksame kosmetische oder dermatologische Zubereitung, **dadurch gekennzeichnet, daß** sie
(a) PEG-30-Dipolyhydroxystearat,
(b) mindestens ein Bis-Resorcinyltriazinderivat,
(c) mindestens ein Dibenzoylmethanderivat und
(d) Titandioxidpartikel
enthält.

2. Zubereitung nach Anspruch 1, **dadurch gekennzeichnet, daß** sie weniger als 1,5 Gew.-%, bezogen auf das Gesamtgewicht der Zubereitung, an Wachsen enthält.

3. Zubereitung nach einem der vorhergehenden Ansprüche, **dadurch gekennzeichnet, daß** sie weniger als 1 Gew.-%, bezogen auf das Gesamtgewicht der Zubereitung, an Wachsen enthält.

4. Zubereitung nach einem der vorhergehenden Ansprüche, **dadurch gekennzeichnet, daß** sie keine weiteren Emulgatoren enthält.

5. Zubereitung nach einem der vorhergehenden Ansprüche, **dadurch gekennzeichnet, daß** der Ölphasenanteil mehr als 35 Gew.-% (bezogen auf das Gesamtgewicht der Zubereitung) beträgt.

6. Zubereitung nach einem der vorhergehenden Ansprüche, **dadurch gekennzeichnet, daß** sie als Bis-Resorcinyltriazinderivat das 2,4-Bis-{[4-(2-Ethyl-hexyloxy)-2-hydroxy]-phenyl}-6-(4-methoxyphenyl)-1,3,5-triazin enthält.

7. Zubereitung nach einem der vorhergehenden Ansprüche, **dadurch gekennzeichnet, daß** sie als Dibenzoylmethanderivat das 4-(tert.-Butyl)-4'-methoxydibenzoylmethan enthält.
